# EUROPÄISCHE PATENTSCHRIFT

(11) **EP 0 313 600 B1**
(45) Veröffentlichungstag und Bekanntmachung des Hinweises auf die Patenterteilung: **02.03.1994**
(21) Anmeldenummer: 88903737.0
(22) Anmeldetag: 05.05.1988
(51) Int. Cl.: C12Q 1/56, G01N 33/86

(54) **VERFAHREN ZUR QUANTITATIVEN BESTIMMUNG VON FUNKTION UND ANTIGENER KONZENTRATION EINES IN EINER BIOLOGISCHEN FLÜSSIGKEIT ENTHALTENEN PLASMINOGENAKTIVATORS**
PROCESS FOR THE QUANTITATIVE DETERMINATION OF THE FUNCTION AND ANTIGENIC CONCENTRATION OF A PLASMINOGENACTIVATOR CONTAINED IN A BIOLOGICAL LIQUID
PROCEDE POUR LA DETERMINATION QUANTITATIVE DE LA FONCTION ET DE LA CONCENTRATION ANTIGENIQUE D'UN ACTIVATEUR DU PLASMINOGENE CONTENU DANS UN FLUIDE BIOLOGIQUE

(30) Priorität: 05.05.1987 AT 1127/87
(43) Veröffentlichungstag der Anmeldung: 03.05.1989
(73) Patentinhaber: BERBI Gesellschaft m.b.H., A-1010 Wien (AT)
(72) Erfinder: Binder, Bernd, Dr., A-1010 Wien (AT)
(74) Vertreter: Piso, Eberhard, Dr.
(86) Internationale Anmeldenummer: AT8800027
(87) Internationale Veröffentlichungsnummer: WO8808883

(56) Entgegenhaltungen:
- EP-A- 0 094 720
- EP-A- 0 141 263
- WO-A-60/1411
- BE-A- 896 253
- US-A- 4 629 694
- CHEMICAL ABSTRACTS, Band 104, Nr. 21, 26. Mai 1986, Columbus, OH (US); C. KORNINGER et al., Seite 251, Nr. 182106k#

## Beschreibung

Die Erfindung betrifft ein Verfahren zur quantitativen Bestimmung von enzymatischer Funktion und antigener Konzentration eines in einer biologischen Flüssigkeit enthaltenen Plasminogenaktivators.

Die Konzentration biologisch bedeutsamer Substanzen in verschiedenen Flüssigkeiten wie Blut, Harn, Liquor oder anderen, wird üblicherweise mittels zweier voneinander völlig unterschiedlicher Methoden unabhängig voneinander bestimmt: Einerseits wird mittels verschiedenster immunologischer Verfahren die Konzentration dieser Substanz erfaßt (antigene Konzentration); solche Verfahren beinhalten den Radioimmunoassay (Travis, J.C., Clinical Radioimmunoassay, State of the Art, Radioassay, Ligand assay Publishers, Anaheim CA 1980), den Enzyme-linked-immunosorbent-assay (Nakamura, R.M., Dito, W.R., Tucker III, E.S., Immunoassays in the Clinical Laboratory, Laboratory and Research Methods in Biology and Medicine, Vol. 3, Alan R. Liss Inc., New York, 1979; Voller, A., Bidwell, D.E., Bartlett, A., The Enzyme Linked Immunosorbent Assay - ELISA, The Authors, 1979, London; Langone, J.J., Van Vunakis, H., Methods in Enzymology, Immunochemical Techniques Part E, 1983, Academic Press; ferner Schuurs et al., United States Patent No. 29,169 und Schuurs et al., United States Patent No. 4,016,043) oder grobe Verfahren (Nakamura, R.M., Dito, W.R., Tucker III, E.S., Immunoassays in the Clinical Laboratory, Laboratory and Research Methods in Biology and Medicine, Vol. 3, Alan R. Liss Inc., New York, 1979; Langone, J.J., Van Vunakis, H., Methods in Enzymology, Immunochemical Techniques Part B, 1981, Academic Press) wie die Immunnephelometrie, die radiale Immunodiffusion oder andere geeignete Immunpräzipitationsverfahren. Mit Hilfe immunologischer Bestimmungen können zwar Konzentrationen einer Substanz erfaßt werden, es ist jedoch unmöglich, auf die Funktion dieser so erfaßten Substanz zurückzuschließen. Es hat sich nämlich öfters gezeigt, daß in verschiedenen Fällen bei einer normalen immunologisch bestimmbaren Konzentration die Funktion dieses Moleküls nicht derjenigen der immunologisch bestimmten entsprach und oft weit unter dem Normalwert lag. Bei solchen Molekülen handelt es sich um Eiweißkörper, denen eine bestimmte enzymatische Funktion oder eine Funktion als Träger oder Cofaktor oder ähnliches in Körperflüssigkeit zukommt und deren Funktion durch Veränderung an bestimmten Molekülteilen beeinträchtigt wird oder welche durch Bindung an Inhibitoren gehemmt wird.

Abgesehen von den allgemein oben erwähnten Methoden sind bereits einige spezifische Methoden im Bereich der hier gewählten Plasminogenaktivatoren zur Bestimmung der immunologischen Funktion publiziert worden; so etwa in der BE-A-896 253, in der monoklonale Antikörper gegen die Urokinase beschrieben und eine Anwendung dieser Antikörper zur Bestimmung der immunologischen Konzentration der Urokinase angegeben werden. In analoger Weise ist auch eine Bestimmung des Gewebeplasminogenaktivators (t-PA) bekannt, wie sie in Chemical Abstracts 104 (1986): 182106k beschrieben ist, wobei ausschließlich eine Methode zur Bestimmung der antigenen Konzentration des Gewebeplasminogenaktivators unter Zuhilfenahme geeigneter Antikörper beschrieben wird. Auch in der WO-A-86/01411, in welcher ebenfalls Anti-urokinase-Antikörper hinsichtlich ihrer Funktion und ihrer Verwendung angegeben werden, wird eine Doppel-Antikörper-Sandwichmethode zur Bestimmung des Antigens der Urokinase sowie auch deren Vorstufe beschrieben.

Die zweite Möglichkeit, die Konzentration einer bestimmten Substanz in Körperflüssigkeiten zu bestimmen, ist die Erfassung der Funktion dieser Substanz (Bergmeyer, H.U., Methods of Enzymatic Analysis, Vol. 5 Verlag Chemie, 1984). Hierbei werden verschiedene Systeme angewandt, die meist auf einer Enzymreaktion aufgebaut sind und die die enzymatische Aktivität der gesuchten Substanz messen (Lorand, L., Proteolytic Enzymes, Methods in Enzymology, Band 45, 1976, Academic Press). Durch die Wahl spezifisch geeigneter Substrate (z.B. hochmolekulare natürliche Substrate, welche durch Enzyme umgewandelt werden, wie das Fibrinogen für sein Enzym Thrombin, das Fibrin als Substrat für Plasmin, das Plasminogen als Substrat für Plasminogenaktivatoren, das Kininogen als Substrat für Kallikrein, aber auch niedermolekulare Substrate, welche die für das jeweilige Enzym spezifischen Peptidbindungen enthalten und deren Spaltung z.B. zur Entwicklung einer Farbreaktion führt) und geeigneter Bestimmungskriterien (hierbei werden im Falle der Bildung von aktiven Enzymen im ersten Schritt die jeweils entstandenen Enzyme gemessen bzw. im Falle von niedermolekularen Substraten die färbigen Produkte bzw. neuentstandene oder verlorengegangene Eigenschaften von Substraten, wie etwa die Fähigkeit, sich an bestimmte Substanzen zu binden; als Beispiel für letzteres kann die Bindung des aktiven Plasminogenaktivatorinhibitors an sein Bindungsprotein gelten, wobei bei Inaktivierung des Plasminogenaktivator-Inhibitors diese Bindungseigenschaft verlorengeht; weitere Beispiele für diese Bestimmungsmethoden sind ferner Bindung an spezifische Inhibitoren, Transportproteine oder andere Bindungsproteine, die entsprechend markiert werden können und durch eine gekoppelte Reaktion nachgewiesen werden können) ist es hierbei möglich, eine relativ hohe Spezifität für die bestimmte Substanz zu erhalten; es ist jedoch unmöglich, einen gemessenen spezifischen Wert einer Substanz zuzuordnen, da üblicherweise eine Vielzahl von Enzymen oder anderen Substanzen eine gleiche oder ähnliche Reaktion an denselben Substraten allerdings mit unterschiedlicher Geschwindigkeit katalysieren. Als Beispiel diene in etwa die Aktivierung des Plasminogens zu Plasmin durch Plasminogenaktivatoren, wobei zumindest zwei hauptsächliche Plasminogenaktivatoren in Körperflüssigkeiten vorhanden sind, die jeweils die selbe Bindung im Plasminogen spalten, wodurch ein identisches Plasmin gebildet wird. Mittels der funktionellen Bestimmungsmethode ist es daher nicht möglich, die Quantifizierung einer bestimmten Substanz vorzunehmen; es ist meist nur möglich, einen funktionellen Defekt in einem gesamten System nachzuweisen.

Auch die in der EP-A-94 720 geoffenbarte Methode verwendet ohne zwischen verschiedenen Formen von Plasminogenaktivatoren zu unterscheiden im wesentlichen die Spaltprodukte des Fibrinogens zur Stimulierung der Aktivität des Gewebeplasminogenaktivators, um die geringe intrinsische Aktivität des Gewebeplasminogenaktivators mittels des angegebenen funktionellen Tests meßbar zu machen.

Mit Hilfe der vorliegenden Erfindung ist es im Gegensatz zu den beiden oben beschriebenen Methodengruppen möglich, sowohl einerseits die funtionelle Aktivität einer genau definierten Substanz (des Plasminogenaktivators) zu messen, als auch im Anschluß daran aus derselben Probe die immunologische Konzentration derselben Substanz quantitativ zu bestimmen. Das Wesen des erfindungsgemäßen Verfahrens liegt darin, zunächst in einem ersten Schritt den gesuchten Plasminogenaktivator spezifisch entweder über eine entsprechende Wechselwirkung mit einem Bindungsprotein oder mit einem spezifischen Antikörper, bevorzugterweise einem monoklonalen Antikörper, an einer Oberfläche zu immobilisieren, den hierbei gebundenen Plasminogenaktivator dann über ein entsprechend geeignetes Plasminogenaktivatorsubstrat (wie z.B. Plasminogenaktivierung und Messung des gebildeten Plasmins mit einem niedermolekularen Plasminsubstrat oder ein niedermolekulares Substrat selbst auf seine Funktion zu überprüfen, diese quantitativ zu bestimmen und im Anschluß daran durch einen geeigneten Antikörper, die gesamte antigene Konzentration des gebundenen Plasminogenaktivators zu messen.

Dieses Verfahren ist gemäß der Erfindung nun dadurch gekennzeichnet, daß der in der biologischen Flüssigkeit enthaltene Plasminogenaktivator immobilisiert wird und dann die enzymatische Funktion durch Zugabe eines spezifischen Plasminogenaktivatorsubstrates gemessen wird, welches in der Folge durch Waschen entfernt wird und daß dann in einem folgenden Schritt die antigene Konzentration des bereits gebundenen Plasminogenaktivators, unabhängig von seiner Funktion, mit Hilfe eines geeigneten Antikörpers bestimmt wird.

Der Vorteil dieser Methode liegt auf folgendem Gebiet: in derselben Probe kann antigene Konzentration und Funktion des gebundenen Plasminogenaktivators überprüft werden.

Die Erfindung besteht ferner darin, daß der in der biologischen Flüssigkeit enthaltene Plasminogenaktivator durch Bindung an eine Plattenoberfläche unter Vermittlung eines polyklonalen oder monoklonalen Antikörpers immobilisiert wird.

Die Erfindung besteht ferner darin, daß man von Plasma oder Harn ausgeht, den darin enthaltenen Plasminogenaktivator immobilisiert und in der ersten Stufe die Plasminbildung durch zugesetztes Plasminogen mißt, und daß man in der zweiten Stufe die antigene Konzentration des gebundenen Plasminogenaktivators mit Hilfe eines monoklonalen Antikörpers, der Peroxydasemarkiert ist, bestimmt.

Die folgenden Beispiele, die nicht einschränkend zu verstehen sind, erläutern mögliche Ausführungsformen des erfindungsgemäßen Verfahrens.

Beispiel 1: System für die Bestimmung von Funktion und Antigen des Gewebeplasminogenaktivators in biologischer Flüssigkeit. Micro-Elisa-Platten werden mit 100 µl pro Napf von 0,035 M Natriumbikarbonat-Puffer, pH 9,6, der 40 µg pro ml 20, 10 oder 5 µg/ml eines gereinigten monoklonalen Antikörpers gegen den Gewebeplasminogenaktivator enthält (MPW1VPA) für 12 Stunden bei 4°C behandelt. Hernach werden diese dreimal mit 300 µl pro Napf PBS, der 0,5 % Tween® 20 enthält, gewaschen. Die verbleibenden Bindungsstellen auf der Polystyren-Platte werden durch Gabe von 100 µl pro Napf 1 % - 25 %-iges bovines Serumalbumin, Lösung in PBS für 4 Stunden bei Raumtemperatur abgesättigt, worauf die Platten neuerlich gewaschen werden, wie oben angegeben. Hierauf können die Platten entweder luftgetrocknet, lyophilisiert oder in geeigneter anderer Weise aufbewahrt werden.

Für die Durchführung der Bestimmungen werden nach einer einmaligen Waschung wie oben mit 100µl einer tPA enthaltenden Lösung, wenn notwendig, verdünnt in PBS, und enthaltend 0.5% bovines Serumalbumin und 0.1% Tween® 80 hinzugeführt. Die Platten werden für zwei Stunden bei 4°C aufbewahrt, wobei die Zeit oder die Temperatur variiert werden können. Die Platten werden hierauf nochmals gewaschen und dann werden 100µl pro Napf einer Lösung, die ein geeignetes Plasminsubstrat, z.B. H-D-Val-Leu-Lys-pNa in einer Konzentration von 0.6mM und 3 verschiedenen Konzentrationen des natürlichen Substrates Glu-Plasminogen (750nM, 375nM und 187nM Endkonzentration) zugefügt und Cyanbromidfragmente des Fibrinogens in einer Konzentration von 75µg/ml. Die Platten werden hierauf 2, 3 oder 5 Stunden bei 37°C in der Dunkelheit inkubiert und hierauf wird die Extinktion bei 405nm gegen 495nm Referenz-Wellenlänge gemessen, wobei ein Zwei-Wellenlängen-Spektrophotometer, z.B. Dynatech Microelisa Reader, verwendet wird. Hiemit wird die Funktion des gebundenen Gewebeplasminogenaktivators überprüft und quantifiziert.

Hierauf werden zur Bestimmung des Antigens die Platten gewaschen wie oben beschrieben und 100µl eines Peroxidase- oder mittels eines geeigneten anderen Enzyms markierten, anderen gegen Gewebeplasminogen-Aktivator gerichtete monoklonalen Antikörpers, z. B. MPW3VPA, Endkonzentration 0.5, 1, 2, und 4ug/ml, zugesetzt. Die Platten werden hierauf für 2 Stunden bei 4°C inkubiert und gewaschen und im Anschluß daran eine Lösung von 1g pro Liter Orthophenylendiamin in 0.11M Natriumphosphat, 0.5M Citrat-Puffer, pH 5.85, zugesetzt, bei dem 0.03% H₂O₂ hinzugefügt werden. 100µl von dieser Lösung werden pro Napf zugegeben. Die Platten werden hierauf für 30 Minuten bei Raumtemperatur in der Dunkelheit inkubiert und im Anschluß daran wird die Reaktion durch den Zusatz von 100µl pro Napf einer 1.5M Schwefelsäure beendet. Die Extinktionen werden bei 495nm und bei 630nm als Referenz-Wellenlänge gemessen, wobei das oben angegebene Photometer verwendet wird.

Als Routineprozedur wurde die Konzentration des ersten Antikörpers mit 10µg/ml, die Konzentration von Glu-Plasminogen von 750nM, die Inkubationszeit für die Plasminbildung 3 Stunden und die Konzentration des zweiten, Peroxidase-markierten Antikörper von 1µg/ml gewählt.

Standardkurven für t-PA Aktivität und tPA Antigen: Gereinigte tPA Präparationen werden in einer Konzentration von 10, 5, 2.5, 1.25, 0.625, 0.3125 und 0.15ng/ml in PBS-Puffer unter Zusatz von 0.1% Tween® 80 und 0.5% BSA hergestellt, wobei auch der internationale Standard in Konzentrationen zwischen 0.075 und 5 Einheiten pro ml verwendet wird. Um nachzuweisen, inwieweit die esterolytische Aktivität unabhängig vom Plasminogen in den Platten vorhanden ist, wurde für das erste Testsystem auch Glu-Plasminogen durch 0.5% bovines Serumalbumin ersetzt. Um zu bestimmen, inwieweit unspezifische Bindung des zweiten Antikörpers das Assays-System beeinflußt, wurde PBS, das 0.5% BSA, 0.01% Tween® enthält, anstelle der t-PA enthaltenden Probe verwendet. EDTA und Natriumcitrat wurden zu den Verdünnungen des gereinigten tPA zugesetzt, um ihren möglichen Einfluß auf die Standardkurve zu überprüfen.

Überprüfung des Effektes von zugesetztem Plasminogensktivator-Inhibitor auf das Assay-System: gereinigter Plasminogenaktivator-Inhibitor wurde in einer Konzentration von einer t-PA hemmenden Einheit pro ml den entsprechenden Verdünnungen des gereinigten t-PA zugesetzt. Andererseits wurde auch der Plasminogenaktivator-Inhibitor in derselben Konzentration einer t-PA-Verdünnung zugesetzt, die schon mit dem ersten Antikörper auf der Platte reagiert hatte. In beiden Fällen wurde die Reaktion des Plasminogenaktivator-Inhibitors mit dem t-PA für 30 Minuten bei 37°C durchgeführt. Als Kontrolle wurde die gleiche Prozedur durchgeführt, wobei PBS enthaltend 0.5% BSA anstelle des Plasminogenaktivator-Inhibitors verwendet wurde. Nachfolgend wurde t-PA-Aktivität und t-PA-Antigen entsprechend bestimmt.

### Recovery-Untersuchungen:

Die Recovery-Untersuchungen wurden durchgeführt, indem eine Standardkurve in einem unverdünnten, gepoolten Plasma, das mit verschiedenen Antikoagulantien erhalten wurde, durchgeführt wurde. Das Plasma war ein Pool von individuellen Plasmaproben, die von 10 gesunden Freiwilligen erhalten wurden. Als Antikoagulantien wurden 0.01 M Natriumcitrat, 0.02M Natrium EDTA und 0.05M Natrium EDTA verwendet. Bei den unterschiedlich antikoagulierten Plasmaproben, welche mit t-PA versetzt wurden, wurde die t-PA Aktivität und t-PA Antigen bestimmt. Von den erhaltenen Resultaten wurde 0.2M Natrium EDTA als Antikoagulans der Wahl ausgesucht. Plasmaproben wurden von gesunden Freiwilligen erhalten und in diesen Plasmaproben wurde ebenfalls die Konzentration des Gewebeplasminogenäktivator-Antigens und der Aktivität vor und nach einer 15-minütigen venösen Stauung bestimmt. Beide Bestimmungen wurden zweifach durchgeführt und die Proben wurden wie oben angegeben mit 0.2M EDTA antikoaguliert.

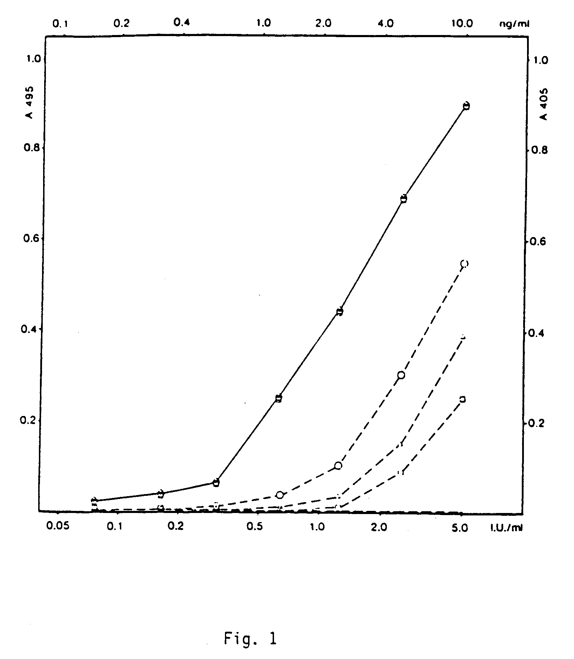

Fig. 1: Eichkurve des Assaysystems zur Bestimmung der t-PA-Aktivität (unterbrochene Linie) und des t-PA Antigen (ausgezogene Linie). Diese wurden mit Puffer, dem Glu-Plasminogen in einer Konzentration von 750nM (offene Kreise), 375nM (offene Dreiecke) und 187nM (offene Vierecke) und 0.5% BSA nach drei Stunden Inkubation durchgeführt. Die funtionelle Aktivität wurde bei 405nm und das Antigen bei 495nm gemessen. Diese sind jeweils gegen die internationalen Einheiten oder ng t-PA, welche zugesetzt wurden, aufgetragen.

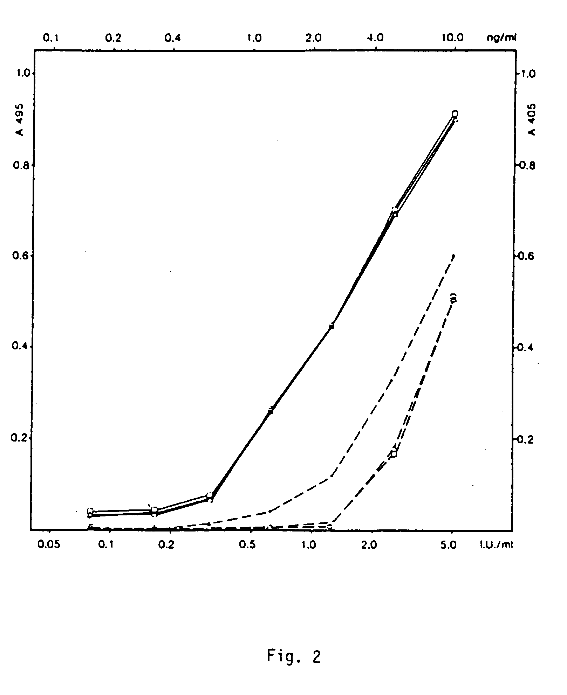

Fig. 2: Dosis-Wirkungs-Kurve für t-PA Aktivität. Unterbrochene Linie: t-PA Antigen, ausgezogene Linie: nach 30 Minuten Vorinkubation bei 37°C mittels t-PA und mit einer Einheit Plasminogenaktivator-Inhibitor bevor (offene Dreiecke) und nach (offene Vierecke) Immobilisierung des t-PA. Als eine Kontrolle wurde der Plasminogenaktivator-Inhibitor durch 0.05% BSA ersetzt. Die funktionelle Aktivität bei 405 nm und 495nm für Antigen sind entsprechend auf der Ordinate aufgetragen gegen die internationalen Einheiten oder ng/ml t-PA auf der Abszisse.

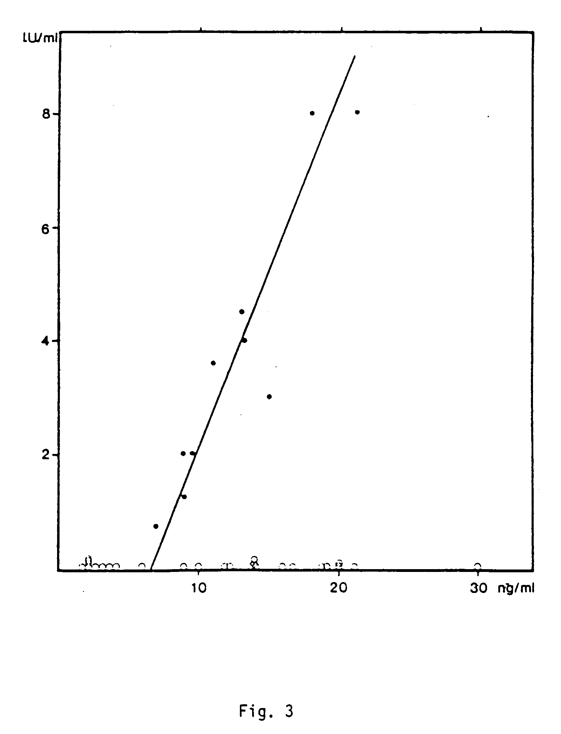

Fig. 3: Korrelation zwischen dem nach venöser Stauung gemessenem t-PA Antigen auf der Abszisse ng/ml und dem nach venöser Stauung t-PA Aktivität auf der Ordinate jeweils pro ml für normale Kontrollen (offene Dreiecke) und von Patienten mit einer verminderten Stauaktivität (offene Kreise).

In der Tabelle sind die Mittelwerte und Standardabweichungen für t-PA Aktivität und t-PA Antigen bei 10 normalen Kontrollen angegeben, und zwar vor und nach venöser Stauung.

| | vor venöser Stauung | nach venöser Stauung |
|---|---|---|
| t-PA Antigen | 2.7 ± 0.5 | 12.6 ± 4.4 |
| t-PA Aktivität | > 0.2 | 3.7 |
| Kontrollgruppe n = 10, mittleres Alter 29.6 ± 8.1 Jahre | | |

Beispiel 2: System für die Bestimmung von Funktion und Antigen des Urokinase-Plasminogenaktivators und der Pro-Urokinase in biologischen Flüssigkeiten. Micro-ELISA-Platten werden mit 100µl pro Napf von 0.035M Natriumbikarbonat-Puffer, pH 9.6, der 20µg eines polyklonalen Ziegen-Antiurokinase-Antikörpers enthält für zwei Stunden bei 4°C behandelt. Danach werden diese dreimal mit 3µl pro Napf PBS, der 0.05% Tween® 20 enthält, gewaschen. Die verbleibenden Bindungsstellen auf der Polystyrenplatte werden hernach 100µl pro Napf, 1%-igen bovines Serumulbumin-Lösung in PBS für 4 Stunden bei Raumtemperatur abgesättigt, worauf die Platten neuerlich gewaschen werden. Hierauf können die Platten entweder luftgetrocknet, lyophilisiert oder in geeigneter anderer Weise aufbewahrt werden.

Für die Durchführung der Bestimmungen werden nach einer einmaligen Waschung die Platten mit 100µl einer u-PA-hältigen Probe, wenn nötig verdünnt in PBS, enthaltend 0.5% BSA, 0.01% Tween® 80 für 2 Stunden bei 37°C inkubiert, wobei Zeit und Temperatur variiert werden können. Die Platten werden hierauf nochmals gewaschen. Hernach werden 100µl pro Napf eines geeigneten Plasminsubstrates, z.B. HDNLE-HHD-Lys-pNA in einer Konzentration von 0.04mM pro Liter sowie das natürliche Substrat Glu-Plasminogen in einer Konzentration von 500nM pro Liter hinzugefügt. Die Platten werden eine Stunde bei 37°C in der Dunkelheit inkubiert. Hernach wird die Absorption bei 405nM mit 495nm Referenzwellenlänge in einem vorzugsweise 2-Wellen-Spektrophotometer gemessen. Wenn in der Probe die Aktivität von Pro-Urokinase gemessen werden soll, muß die Probe zunächst mit einer 0.5µM pro Liter Humanplasminlösung gebunden an Sepharose® für eine Stunde bei 37°C in einer Menge von 100µg/ml behandelt werden. Nach Entfernung der Plasmin-Sepharose kann dann die Aktivität der Prourokinase in gleicher Weise gemessen werden, wobei während der Bindung 10 Einheiten pro ml Aprotinin anwesend sein sollen. Nach der Bestimmung der Funktion werden die Platten neuerlich gewaschen und 100µl pro Napf eines Peroxydase-markierten monoklonalen Antikörpers, z.B. MPW5UK, in einer Konzentration von 1µg pro ml werden hinzugefügt und die Platen für 2 Stunden bei 37°C inkubiert. Im Anschluß daran werden die Platten gewaschen und eine Lösung von 1mg pro Liter Orthophenylendiamin in 0.11 Mol pro Liter Natriumphosphat, 0.05µl Citratpuffer, pH 5.85, der 0.03% H₂O₂ enthält, hinzugefügt in einer Menge von 100µl. Die Platten werden für 15 Minuten bei Raumtemperatur in der Dunkelheit inkubiert und die Reaktion beendet durch Hinzusetzung von 100ug pro Napf 1.5 Mol pro Liter Schwefelsäure. Die Absorption wird bei 495nm mit 540nm Referenzwellenlänge gemessen, am besten in einem Zwei-Wellenlängen-Spektrophotometer. Anstelle von Orthophenylendiamin kann auch ein geeignetes anderes Peroxydasesubstrat verwendet werden bzw. auch die Markierung des zweiten Antikörpers mit einem anderen Enzym kann in entsprechender Weise erfolgen.

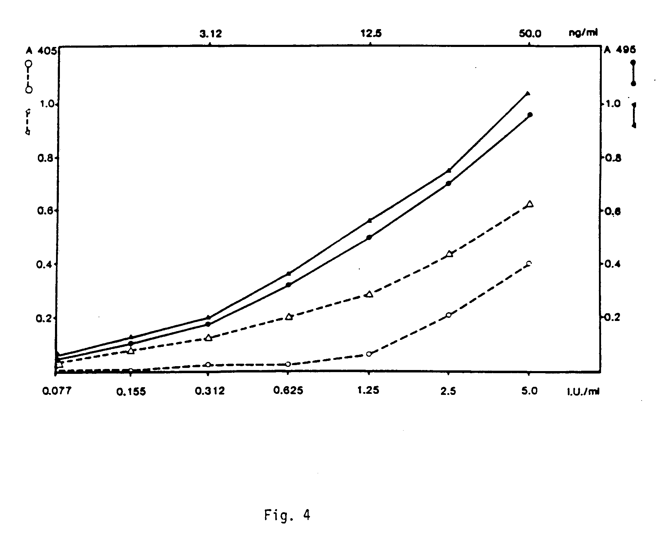

Standardkurven für Urokinase werden dadurch erhalten, daß Konzentrationen von 5, 2.5, 1.25, 0.625, 0.312 und 0.155 und 0.0677 Internationale Einheiten pro ml Urokinase zugesetzt werden und in dem entsprechenden Testsystem bestimmt werden. Die Ergebnisse für Urokinase in Puffer und Plasma sind in Fig. 4 angegeben: Absorptionswerte in dem Aktivitätsassay (offene Symbole, bei 405nm gemessen) für u-PA in Puffer (Dreieck) und Plasma (Kreis) gefolgt bei der Bestimmung des Antigenspiegels (geschlossene Symbole, Absorption bei 495nm) in Puffer (Dreieck) und Plasma (Kreis). Während die antigene Konzentration der zugefügten Urokinase in Puffer und Plasma identisch ist, kommt es im Plasma zu einer deutlichen Verminderung der Aktivität der zugesetzten Urokinase, welche auf Bindung eines spezifischen Plasminogenaktivator-Inhibitors an Urokinase zurückzuführen ist. Aus diesen Daten kann eine Plasma-Recovery der zugesetzten Urokinase von 88% für den Antigen-Assay errechnet werden, wenn die Konzentration von Natrium-EDTA 15mMol pro Liter, jene von Benzamidin-Hydrochlorid 20mMol pro Liter und die von Aprotinin 10 U pro ml in der Probe mindestens erreicht wird.

Bei der Untersuchung von 10 Normalplasmaproben wurde keine frei u-PA Aktivität nachgewiesen, während der mittlere Gehalt an Urokinase-Antigen 1.88 ± 0.61ng/ml war, die Intereassay-Variation für den Aktivitäts- und Antigen-Assay war 1.9 und 6.8% und die Intraassay-Variation 9.6 und 4.8%.

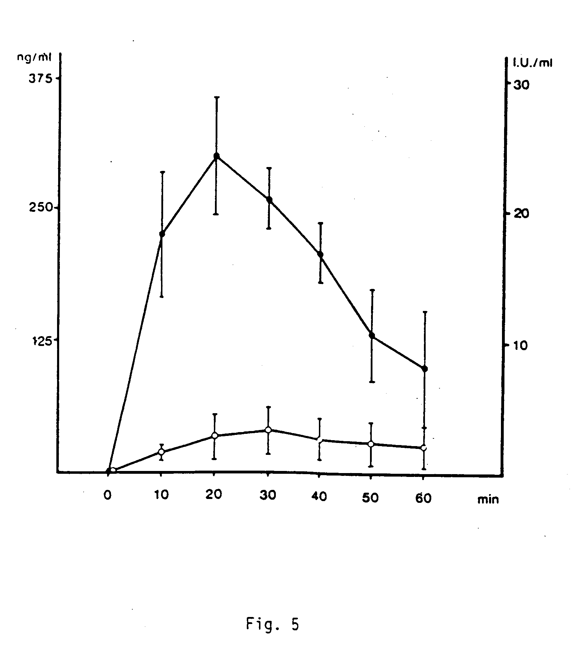

Bei der Untersuchung von Urokinasespiegeln bei Patienten mit Urokinase-Behandlung zum Zwecke der Auflösung von Thrombosen kann ein Anstieg des Urokinase-Antigens unmittelbar am Ende der initialen Urokinasegabe ermittelt werden. Ein typisches Beispiel für 4 Patienten ist in der Fig. 5 angegeben.

Fig. 5: Urokinase-Antigen (geschlossener Kreis) und Aktivität (offener Kreis) bei 4 Patienten während thrombolytischer Therapie mit Urokinase. Urokinase wurde infundiert in einer Dosierung von 7.500 ± 1.600 Einheiten pro kg Körpergewicht innerhalb von 25 Minuten gefolgt von einer Infusion von 1900 ± 400 Internationale Einheiten pro kg Körpergewicht und Stunde.

## Patentansprüche

1. Verfahren zur quantitativen Bestimmung von enzymatischer Funktion und antigener Konzentration eines in einer biologischen Flüssigkeit enthaltenen Plasminogenaktivators, dadurch gekennzeichnet, daß der in der biologischen Flüssigkeit enthaltene Plasminogenaktivator immobilisiert wird und dann die enzymatische Funktion durch Zugabe eines spezifischen Plasminogenaktivatorsubstrates gemessen wird, welches in der Folge durch Waschen entfernt wird, und daß dann in einem folgenden Schritt die antigene Konzentration des bereits gebundenen Plasminogenaktivators, unabhängig von seiner Funktion, mit Hilfe eines geeigneten Antikörpers bestimmt wird.

2. Verfahren nach Anspruch 1, dadurch gekennzeichnet, daß der in der biologischen Flüssigkeit enthaltene Plasminogenaktivator durch Bindung an eine Plattenoberfläche unter Vermittlung eines polyklonalen oder monoklonalen Antikörpers immobilisiert wird.

3. Verfahren nach Anspruch 1 oder 2, dadurch gekennzeichnet, daß man von Plasma oder Harn ausgeht, den darin enthaltenen Plasminogenaktivator immobilisiert und in der ersten Stufe die Plasminbildung durch zugesetztes Plasminogen mißt, und daß man in der zweiten Stufe die antigene Konzentration des gebundenen Plasminogenaktivators mit Hilfe eines monoklonalen Antikörpers, der Peroxydase-markiert ist, bestimmt.

## Claims

1. Process for the quantitative determination of enzymatic function and antigenic concentration of a plasminogen activator contained in a biological liquid, characterised in that the plasminogen activator contained in the biological liquid is immobilised and then the enzymatic function is measured by the addition of a specific plasminogen activator substrate, which is subsequently removed by washing, and that then in a following step the antigenic concentration of the already bound plasminogen activator is determined, independently of its function, with the aid of a suitable antibody.

2. Process according to claim 1 characterised in that the plasminogen activator contained in the biological liquid is immobilised by bonding to a plate surface via the intermediation of a polyclonal or monoclonal antibody.

3. Process according to claim 1 or 2 characterised in that starting from plasma or urine the plasminogen activator contained therein is immobilised and in the first stage the plasmin formation by added plasminogen is omitted, and that in the second stage the antigenic concentration of the bound plasminogen activator is determined with the aid of a monoclonal antibody which is peroxidase marked.

## Revendications

1. Procédé de détermination quantitative de la fonction enzymatique et de la concentration en antigènes d'un activateur plasminogène contenu dans un liquide biologique, caractérisé en ce que l'activateur plasminogène contenu dans le liquide biologique est immobilisé et la fonction enzymatique est mesurée ensuite par addition d'un substrat d'activateur plasminogène spécifique, éliminé ensuite par lavage, et en ce que l'on détermine ensuite, lors d'une étape subséquente, la concentration en antigène de l'activateur plasminogène déjà combiné, indépendamment de sa fonction, à l'aide d'un anticorps approprié.

2. Procédé selon la revendication 1, caractérisé en ce que l'activateur plasminogène contenu dans le liquide biologique est immobilisé par liaison sur une surface de plaque, avec utilisation d'un anticorps polyclonal ou monoclonal.

3. Procédé selon la revendication 1 ou 2, caractérisé en ce que l'on part de plasma ou d'urine, on immobilise l'activateur plasminogène y étant contenu et l'on mesure en une première étape la formation de plasmine par addition de plasminogène, et en ce que, dans une deuxième étape, on détermine la concentration en antigène de l'activateur de plasminogène lié à l'aide d'un anticorps monoclonal, marqué par une peroxydase.
